# EUROPÄISCHE PATENTSCHRIFT

(11) **EP 1 524 012 B1**
(45) Veröffentlichungstag und Bekanntmachung des Hinweises auf die Patenterteilung: **10.09.2008**
(21) Anmeldenummer: 05000103.1
(22) Anmeldetag: 23.05.2001
(51) Int. Cl.: A61N 5/10

(54) **Vorrichtung zum Plazieren eines Patienten**
Device for positioning a patient
Dispositif de mise en place d'un patient

(30) Priorität: 26.05.2000 DE 10025913
(43) Veröffentlichungstag der Anmeldung: 20.04.2005
(62) Teilanmeldung aus: 01960240.8
(73) Patentinhaber: Gesellschaft für Schwerionenforschung mbH, 64291 Darmstadt (DE)
(72) Erfinder: Schardt, Dieter, 64291 Darmstadt (DE); Heeg, Peter, 64291 Darmstadt (DE)
(74) Vertreter: Boeters, Hans Dietrich

(56) Entgegenhaltungen:
- EP-A- 0 986 070
- WO-A-99/53997
- WO-A-99/53998
- DE-A1- 3 502 776
- FR-A- 2 748 650
- US-A- 4 583 537
- US-A- 5 250 019
- US-A- 5 668 371
- PATENT ABSTRACTS OF JAPAN Bd. 1997, Nr. 02, 28. Februar 1997 (1997-02-28) & JP 08 266650 A (MITSUBISHI ELECTRIC CORP), 15. Oktober 1996 (1996-10-15)
- PATENT ABSTRACTS OF JAPAN Bd. 017, Nr. 489 (C-1106), 6. September 1993 (1993-09-06) & JP 05 123409 A (NAGASHIMA IKA KIKAI KK), 21. Mai 1993 (1993-05-21)
- PATENT ABSTRACTS OF JAPAN Bd. 017, Nr. 462 (C-1101), 24. August 1993 (1993-08-24) & JP 05 111516 A (NAGASHIMA IKA KIKAI KK), 7. Mai 1993 (1993-05-07)

## Beschreibung

Die Erfindung betrifft eine Vorrichtung zum Plazieren eines Tumor-Patienten mit einem Tumor im Kopf-Halsbereich.

Bekannte Schwerionentherapieräume verfügen über Patientenliegen, auf denen der Patient im Kopf-Halsbereich mittels einer Bestrahlungsmaske fixiert wird und die Patientenliege in bezug auf ein horizontales Strahlrohr für den Schwerionentherapiestrahl ausgerichtet wird. Derartige bekannte Einrichtungen erlauben Einstrahl- bzw. Einschußrichtungen in eine Frontalebene des Patientenkopfes, was in begrenzten Fällen zufriedenstellende Dosisverteilungen ermöglicht. Dazu wird durch zwei schnell arbeitende Ablenkmagnete in horizontaler und vertikaler Richtung quer zum Ionenstrahl der Ionenstrahl über den Tumorquerschnitt geführt, wobei die Eindringtiefe des Schwerionenstrahls durch Verändern der Schwerionenstrahlenergie und die Bestrahlungsmenge durch Einstellen der Schwerionendosis in einem Bestrahlungsplan in Abhängigkeit von Größe und räumlicher Erstreckung des Tumorgewebes festgelegt ist. Während der Bestrahlung wird der Bestrahlungsvorgang durch eine im Bestrahlungsraum installierte PET-Kamera überwacht.

Jedoch durch die häufige Konstellation von Tumoren, die direkt an Risikoorgane angrenzen wie an den Hirnstamm, an den Sehnerv, an das Chiasma oder an die Augen, ist es mit dem nur einen Freiheitsgrad, der durch das Drehen der Patientenliege um eine vertikale Achse ermöglicht wird, nicht immer möglich, alle Risikoorgane des Kopf-Halsbereiches eines Patienten ausreichend zu schonen. Deshalb kann nicht jeder Bestrahlungsplan, bei dem die Belastung der einzelnen Risikoorgane entsprechend einem Dosisvolumenhistogramm noch tolerabel wäre, in der Praxis angewendet werden, da in solchen Fällen kein risikoloser Einstrahlwinkel oder Einschußwinkel für den Ionenstrahl im Kopf-Halsbereich mit absoluter Sicherheit gegeben ist.

Die Anwendung des Schwerionenstrahls wird auch deshalb eingeschränkt, weil Ionen nach dem Passieren von sehr inhomogenem Gewebe unmittelbar vor einem Risikoorgan stoppen müßten. Eine geringfügige laterale Fehlpositionierung würde zu einer beträchtlichen Reichweitenveränderung führen und damit zu einer Fehldosierung im Risikoorgan. Deshalb sind mit den eingeschränkten Möglichkeiten einer Patientenliege auch nur eingeschränkte Möglichkeiten der Behandlung von Tumoren im Kopf-Halsbereich möglich.

Aufgabe der Erfindung ist es, eine Vorrichtung anzugeben, die alternativ zu einer Patientenliege mindestens einen weiteren Freiheitsgrad für die Ausrichtung des Patientenkopfes ermöglicht.

Bekannte Lösungen sind dazu, eine Ionenstrahlgantry aufzubauen, in die eine Patientenliege eingeschoben wird und der Ionenstrahl in einem Trommelgestell geführt wird, so daß er aus jeder Raumrichtung den Patienten bestrahlen kann. Derartige kostenintensive Lösungen sind jedoch für einen Ionentherapieraum ungeeignet, wenn der Ionenstrahl lediglich aus einer unveränderbaren Raumrichtung in bezug auf die Raumkoordinaten horizontal strahlt. Auch die geringfügige Auslenkung des Ionenstrahls beim schnellen Abtasten eines Tumorquerschnitts kann dieses Problem für einen derartig ausgestatteten Ionenstrahltherapieraum nicht lösen. Auch die räumlichen Dimensionen einer Gantry, die sich über mehrere Gebäudestockwerke ausdehnt, sind für begrenzte Ionentherapieräume ungeeignet.

Gelöst wird diese Aufgabe mit den unabhängigen Ansprüchen. Bevorzugte Weiterbildungen der Erfindung werden in den abhängigen Ansprüchen offenbart.

Erfindungsgemäß weist die Vorrichtung zum Plazieren eines Tumor-Patienten alternativ zu einer Patientenliege eine Vorrichtung auf, die den Patienten in sitzender Haltung fixiert. Diese Vorrichtung hat Mechanismen, die den Tumor des Patienten unter den Bewegungsfreiheitsgraden der Vorrichtung in dem Isozentrum des Ionenstrahls hält. Das Halten des Tumors in dem Isozentrum des Ionenstrahls hat den Vorteil, daß alle verfügbaren Freiheitsgrade, die eine sitzende Position des Patienten bietet, sich lediglich auf den Winkel, unter dem das Tumorgewebe beschossen werden kann, auswirken, nicht aber auf die Fixierung des Patienten in den drei Raumkoordinaten X, Y und Z, das heißt, der Ursprung des karthesischen Koordinatensystems ist gleichzeitig Zentrum des Tumors und Schnittpunkt mit dem Ionenstrahl, und diese Konstellation wird trotz der Veränderung und Einstellung des Winkels über die zusätzlichen Drehbewegungsfreiheitsgrade der Vorrichtung für eine sitzende Position des Patienten beibehalten.

Die Vorrichtung weist als weiteren Bewegungsfreiheitsgrad eine Kippbewegung um mindestens eine horizontale Achse auf, die den Ionenstrahl im Isozentrum schneidet. Eine derartige Kippbewegung ist für die herkömmlichen Patientenliegen nicht vorgesehen, so daß mit dieser Kippbewegung der Vorrichtung bisher nicht behandelbare Patienten behandelt werden können.

Die Vorrichtung weist auch einen Bewegungsfreiheitsgrad einer Drehbewegung um eine vertikale Achse auf, die den Ionenstrahl im Isozentrum schneidet. Mit diesem Bewegungsfreiheitsgrad der Vorrichtung ist die Vorrichtung an die Möglichkeiten einer Patientenliege angepaßt, die nur diesen einen Drehbewegungsfreiheitsgrad aufweist, so daß für eine Patientenliege vorgesehene Behandlungspläne in einfacher Weise in Behandlungspläne für die neue Vorrichtung umgerechnet oder transformiert werden können.

Die Vorrichtung weist die Bewegungsfreiheitsgrade der drei Translationen in den stereotaktischen Koordinaten X, Y und Z auf. Die räumliche Anordnung der Antriebe für die unterschiedlichen Freiheitsgrade der unterschiedlichen Ausführungsformen der Erfindung sind derart aufeinander abgestimmt, daß die Antriebe für die translatorischen Bewegungen örtlich über den Antrieben für die Drehbewegungen angeordnet sind. Erst diese örtliche Anordnung versetzt die Vorrichtung in die Lage, die Rotationsfreiheitsgrade nutzen zu können und gleichzeitig die Tumorposition im Isozentrum beizubehalten. Damit wird in vorteilhafter Weise auch die Einstellung der Vorrichtung in bezug auf das Isozentrum erleichtert, indem zunächst die Zielkoordinaten für die Bestrahlung eines Tumors im Kopf-Halsbereich durch die drei Translationsbewegungen eingestellt wird und anschließend eine isozentrische Rotation und/oder eine isozentrische Kippung der Vorrichtung durchgeführt wird.

Dazu sind in einer bevorzugten Ausführungsform der Erfindung die Antriebe für die Drehbewegungen um eine horizontale und eine vertikale Achse mit ihren Schnittpunkten im Isozentrum des Ionenstrahls unter der Sitzposition eines Patienten und räumlich unter den translatorischen Antrieben angeordnet.

In einer weiteren bevorzugten Ausführungsform der Erfindung weist die Vorrichtung zur Drehung oder Kippung um eine horizontale Achse Bogenführungen unter der Sitz- und/oder Liegefläche auf. Die Liegefläche kommt jedoch nur dann zum Tragen, wenn in einer bevorzugten Ausführungsform der Erfindung die Sitzposition des Patienten in eine Liegeposition verstellbar ist. Dieses erfordert jedoch einen hohen technischen Aufwand, wenn gleichzeitig die horizontale und vertikale Drehbewegung im Isozentrum für den Kopf-Halsbereich eines Patienten beibehalten werden soll.

In einer bevorzugten Ausführungsform der Erfindung sind alle verstellbaren Bewegungsfreiheitsgrade elektromotorisch einstellbar. Derartige elektromotorische Antriebe haben den Vorteil, daß sie sowohl die translatorischen Einstellungen als auch die rotatorischen Einstellungen mit höchster Präzision durchführen können, so daß in translatorischer Richtung eine Genauigkeit von unter 0,5 mm erreichbar ist und in rotatorischer Richtung eine Winkelabweichung unter 0,1° einstellbar wird.

In einer weiteren bevorzugten Ausführungsform der Vorrichtung weist diese zum Verschieben in X-, Y- und Z-Richtung, nämlich den drei Translationen der stereotaktischen Koordinaten und zum Verdrehen um eine horizontale und eine vertikale Achse als Antriebsaggregate Schrittmotoren mit Positionsmessern, Endschaltern und elektronischen Steuermodulen auf. Die Schrittmotoren haben den Vorteil, daß sie über elektronische Steuermodule digital angesteuert werden können und in Schritten der vorgegebenen Genauigkeit die Translations- und die Rotationsbewegungen der Vorrichtung durchführen. Die Endschalter bieten eine zusätzliche Sicherheit gegen extreme Winkel und extreme seitliche Auslenkungen der Einstellparameter sowie gegen Fehlinterpretationen des Bestrahlungsplans durch elektronische Steuermodule.

In einer bevorzugten Weiterbildung der Erfindung sind die Antriebe für translatorische Verschiebungen der Vorrichtung außerhalb einer unmittelbaren Sitzposition der Vorrichtung angeordnet. Dies hat den Vorteil, daß die Sitzposition so niedrig wie möglich angeordnet werden kann, beispielsweise wenn der translatorische Antrieb für die Höheneinstellung in Z-Richtung im Bereich der Sitzlehne angeordnet ist.

Insbesondere ist vorzugsweise die Vorrichtung mit einer Not-Aus-Schaltungsautomatik ausgestattet, um bei offensichtlichen Fehlinterpretationen der Behandlungsposition ein schnelles und automatisches Eingreifen und Korrigieren zu ermöglichen. Dazu ist die Vorrichtung mittels eines Steuerprogramms steuerbar, das einen Kollisionsschutz vorsieht und mit einer Bewegungsgrenz-Überwachungseinrichtung zusammenwirkt.

In einer weiteren bevorzugten Ausführungsform der Erfindung ist eine translatorische Verschiebung der Vorrichtung in Richtung des Ionenstrahls auf Verfahrschienen vorgesehen, wobei ein großer Verfahrweg der Vorrichtung von einer Parkposition in eine Patientenbehandlungsposition vorgesehen ist und eine von den Fahrschienen unabhängige Feineinstellungsvorrichtung in der Patientenbehandlungsposition wirksam wird. Dieses hat den Vorteil, daß die erfindungsgemäße Vorrichtung, die den Patienten in sitzender Position fixiert, kurzfristig in eine Parkposition verschiebbar ist, die den Einsatz einer Patientenliege nicht behindert. In einer weiteren bevorzugten Ausführungsform der Erfindung ist die Positionsgenauigkeit der Vorrichtung in allen translatorisch verstellbaren Freiheitsgraden kleiner oder gleich 0,5 mm, vorzugsweise kleiner gleich 0,1 mm. Diese hohe Positionsgenauigkeit gewährleistet, daß die Vorrichtung exakt im Isozentrum für den Ionenstrahl einstellbar ist und örtliche Abweichungen und damit Fehlbestrahlungen vermieden werden. Deshalb ist die Vorrichtung mit einer Genauigkeit von ±1 bis ±0,5 mm in dem Isozentrum einstellbar. Dazu ist die Vorrichtung vorzugsweise mit einem Positionskontrollgerät im Ionenbehandlungsraum zusammenwirkend verbunden, so daß die Tumorposition im Isozentrum überwacht bleibt. Ein derartiges Positionskontrollgerät ist vorzugsweise eine Röntgenstrahlkamera. Diese Röntgenstrahlkamera vermißt vor und nach der Behandlung exakt die eingestellten translatorischen Positionen und stellt damit sicher, daß die Vorrichtung auch zwischen den Behandlungen präzise arbeitet.

In einer weiteren bevorzugten Ausführungsform der Erfindung weist die Vorrichtung einen Rechner auf, der wahlweise die Zielkoordinaten und Behandlungseinstellungen für eine Plazierung des Patienten in liegender und/oder sitzender Position umrechnet. Durch einen derartigen Rechner ist es vorteilhaft möglich, beide Bestrahlungspositionen aus einer liegenden und aus einer sitzenden Position zu kombinieren und die Bestrahlung unter unterschiedlichen Bestrahlungswinkeln vorzunehmen, so daß das über dem Tumor liegende gesunde Gewebe optimal geschont wird.

Zur Überwachung der Bestrahlung eines Patienten sind vorzugsweise die Kameraköpfe der PET-Kamera um die Ionenstrahlachse drehbar gelagert. Diese drehbare Lagerung ermöglicht es mit ein- und derselben PET-Kamera sowohl die Bestrahlung auf einer Patientenliege als auch auf einer Vorrichtung mit Sitzposition des Patienten zu überwachen.

Somit ist in einer bevorzugten Ausführungsform der Vorrichtung eine kombinierte Bestrahlung eines Patienten in Verbindung mit einer Patientenliege und der erfindungsgemäßen Vorrichtung in sitzender und liegender Haltung möglich.

Zur Anpassung der Körpergröße des Patienten in bezug auf das Isozentrum des Ionenstrahls weist die Vorrichtung eine ausreichend verstellbare Höheneinstellung in Z-Richtung auf. Dazu hat die Höhenverstelleinrichtung einen Verfahrbereich von ±100 bis ±500 mm, vorzugsweise von ±200 bis ±300 mm. Diese Höhenverstelleinrichtung kann mit einer Verfahrgeschwindigkeit von 1 bis 15 mm/s, vorzugsweise von 2 bis 5 mm/s betrieben werden, wobei die hohen Verfahrgeschwindigkeiten ohne Patienten ausgeführt werden, während die langsameren Verfahrgeschwindigkeiten mit positioniertem Patienten auszuführen sind.

In der Patientenbehandlungsposition weist vorzugsweise die Antriebsvorrichtung in den horizontalen Translationsverschiebungen in X- und Y-Richtung einen Verfahrbereich von 100 bis 200 mm, vorzugsweise von 120 bis 150 mm auf, die mit einer Verfahrgeschwindigkeit zwischen 5 und 200 mm/s und vorzugsweise zwischen 8 und 10 mm/s liegt.

Vorzugsweise ist die Drehung um eine horizontale Achse auf eine bevorzugte Kippbewegung begrenzt, die mit einem Kippbereich von ±30°, vorzugsweise ±20° arbeitet, wobei die Geschwindigkeit der Kippbewegung zwischen 0,5 bis 1 °/s, vorzugsweise zwischen 0,6 bis 0,8 °/s liegt. Auch hier werden bei Anwesenheit eines fixierten sitzenden Patienten die niedrigeren Geschwindigkeiten der Kippbewegung eingesetzt.

Der Drehbereich um eine vertikale Achse ist nicht begrenzt und kann einen vollständigen Kreis von 0 bis 360° aufweisen. Bei einer sitzenden Haltung des Patienten zur Ionenbehandlung des Patienten im Kopf-Halsbereich kann diese Drehbewegung in vorteilhafter Weise auf engerem Raum durchgeführt werden als mit einer Patientenliege. Dabei wird in einer bevorzugten Ausführungsform der Erfindung eine Drehgeschwindigkeit um die vertikale Achse zwischen 1 bis 10 °/s, vorzugsweise zwischen 3 bis 6 °/s eingesetzt.

Ein bevorzugtes Verfahren zur Behandlung eines Tumors eines Patienten in einem Kopf- und/oder Halsbereich in einem Schwerionenbehandlungsraum mit einer in bezug auf die Raumkoordinaten festliegenden Ionenstrahlrichtung weist unter Verwendung der erfindungsgemäßen Vorrichtung zur Plazierung eines Tumor-Patienten folgende Verfahrensschritte auf:
- Berechnen eines optimalen Einschußwinkels für den Ionenstrahl (5) durch gesundes Gewebe in Richtung auf den Tumor unter Berücksichtigung von Risikobereichen;
- Verfahren des Patientenstuhls mit fixiertem Patienten aus einer Parkposition in eine Behandlungsposition;
- Einstellen der stereotaktischen Zielpunktkoordinaten durch drei Translationen in X-,Y- und Z-Richtung , so daß der Tumor in dem Isozentrum positioniert wird;
- Einstellen des optimalen Einschußwinkels durch Drehbewegung im Isozentrum um eine horizontale und/oder vertikale Achse;
- Dosierte Bestrahlung des Tumorgewebes unter Schonung des umgebenden Gewebes unter dem berechneten optimalen Einschußwinkel.

Bei einer bevorzugten Durchführung des Verfahrens werden nach Erreichen der Behandlungsposition zunächst die translatorischen Einstellungen in X-, Y- und Z-Richtung vorgenommen, bis der Tumor im Isozentrum des Ionenstrahls angeordnet ist, und danach wird die Drehung um eine horizontale Achse durchgeführt, die mit Hilfe der Bogenführungen unter dem Sitzbereich der Vorrichtung eingestellt wird, und abschließend wird die Dreheinstellung um eine vertikale Achse durchgeführt. Da erfindungsgemäß die Antriebe für die translatorischen Bewegungen zum Ausrichten des Tumors im Isozentrum des Ionenstrahls räumlich über der Bogenführung für die Kippbewegung und dem Antrieb für die Drehbewegung angeordnet sind, und die Achsen der Drehbewegung sich in vorteilhafter Weise im Isozentrum schneiden, ist mit den Drehbewegungen in vorteilhafter Weise keine translatorische Verschiebung des Tumors verbunden.

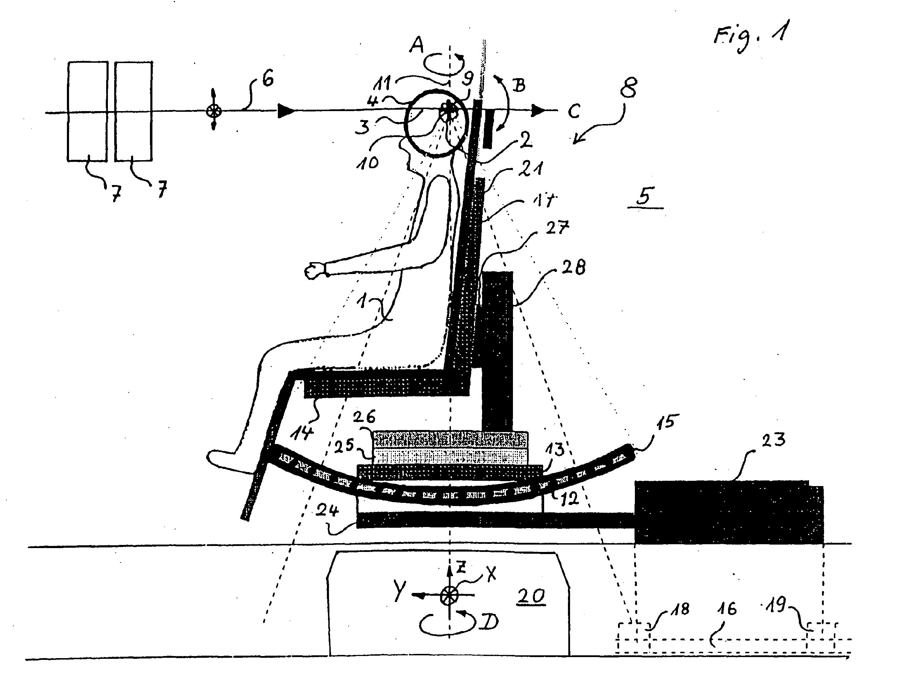

Weitere Merkmale, Vorteile und Eigenschaften der Vorrichtung werden nun anhand eines Ausführungsbeispiels mit Bezug auf Fig. 1 näher beschrieben.

Fig. 1 zeigt eine Vorrichtung 8 zum Plazieren eines Tumor-Patienten 1 mit einem Tumor 2 im Kopf-Halsbereich 3 in einem Schwerionentherapieraum 5 in bezug auf einen Ionenstrahl 6 aus einer unter Raumkoordinaten festliegenden unveränderbaren Richtung C. Der Ionenstrahl 6 kann mittels schneller Ablenkmagnete 7 orthogonal zum Strahl in horizontaler und vertikaler Richtung über den Tumorquerschnitt geführt werden. Die Eindringtiefe des Ionenstrahls 6 kann durch Verändern der Ionenenergie und die Bestrahlungsmenge durch Einstellen der Ionendosis in dem Bestrahlungsraum festgelegt werden.

Als Ionen werden üblicherweise Kohlenstoffionen eingesetzt, jedoch kann in einem derartigen Behandlungsraum auch mit leichten Ionen wie Protonen gearbeitet werden.

Die Bestrahlung des Patienten wird mittels einer PET-Kamera, die hier nicht gezeigt ist, überwacht. Bei dieser Ausführungsform der Erfindung kreuzt sich die Drehbewegung A um eine vertikale Achse 11 und die Drehbewegung B um eine horizontale Achse 10 im Isozentrum 9 des Ionenstrahls 6. Auf dieser Vorrichtung zum Plazieren eines Tumor-Patienten wird der Patient in sitzender Stellung fixiert.

Quergestellt zu der abgebildeten Vorrichtung befindet sich in einer nichtgezeigten Parkposition eine Patientenliege, die üblicherweise für derartige Bestrahlungsräume eingesetzt wird. Eine derartige Patientenliege zur Behandlung von Tumoren im Kopf- und Halsbereich 3 eines Patienten beansprucht einen im Vergleich zu der abgebildeten Vorrichtung wesentlich größeren Drehradius, da der Patient auf einer Patientenliege um das Isozentrum mit dem Tumor im Kopf- oder Halsbereich gedreht werden muß. Die Antriebseinrichtung 20 für eine Patientenliege ist in dieser Ausführungsform unmittelbar unter dem Patientenstuhl 21 angeordnet.

Die translatorischen Richtungen X, Y und Z sind auch bei der Antriebseinheit 20 der Patientenliege vorgesehen, und mit einer Drehbewegungsrichtung D der Patientenliege um eine vertikale Achse 11 kann in begrenzter Form ein Einstrahl- oder Einschußwinkel des Ionenstrahls 6 in der Frontalebene eingestellt werden. Um die nichtgezeigte PET-Kamera sowohl beim Einsatz des Patientenstuhles 21 als auch beim Einsatz der nichtgezeigten Patientenliege verwenden zu können, sind die Kameraköpfe um die Strahlachse C des Ionenstrahls 6 drehbar gelagert. Im Liegebetrieb werden die Kameraköpfe dann vertikal ausgerichtet und im Betrieb mit dem Patientenstuhl werden sie zur Überwachung der Bestrahlung horizontal eingestellt.

Der Patientenstuhl 21 ist auf einer freitragenden Plattform 24, die von einer Vorrichtung 23 gehalten wird, angeordnet. Die Vorrichtung 23 ist mittels Unterbodenführungen 18 und 19 auf Unterbodenverfahrschienen 16 verfahrbar. Die freitragende Plattform 24 kann mit Hilfe der Vorrichtung 23 in eine Parkposition verfahren werden, wenn ein Patient auf einer Patientenliege behandelt werden soll, und wird zur Behandlung in die in Fig. 1 abgebildete Behandlungsposition verfahren, wobei die Plattform 24 über der Antriebseinheit 20 der Liege angeordnet wird. Die Freiheitsgrade zur Einstellung des Patientenstuhls sind von oben nach unten in folgender Reihenfolge räumlich angeordnet:
1. Kippung mit Hilfe der Antriebseinheit 12
2. Drehung mit Hilfe der Antriebseinheit 13
3. Translation in den horizontalen Richtungen X und Y mit den Antriebseinheiten 25 für die X-Richtung und 26 für die Y-Richtung
4. Eine vertikale Translation, deren Antriebseinheit 27 in Z-Richtung an einer Säule 28 befestigt ist, die vertikal auf den translatorischen Antrieben 25 und 26 steht.

Die vertikale Translation dient zur Körpergrößenanpassung des Patienten. Dazu ist die Antriebseinheit 27 für die vertikale Translation mit der Patientenstuhllehne 17 verbunden. Die wesentlichen technischen Daten dieser Ausführungsform der Erfindung zeigt die Tabelle 1.

**Tabelle 1**

| | | |
|---|---|---|
| Translation horizontal: | Verfahrbereich: | ± 120 mm |
| | Geschwindigkeit: | 8,3 mm/s |
| Translation vertikal: | Verfahrbereich: | ± 250 mm |
| | Geschwindigkeit: | 2 mm/s |
| | Geschwindigkeit ohne Patient: | auch 4 mm/s |
| Drehung: | Drehbereich: | 360° |
| | Geschwindigkeit: | 3,3 °/s |
| Kippung: | Kippbereich: | ± 19° |
| | Geschwindigkeit: | 0,75 °/s |
| Genauigkeiten: | Genauigkeit Isozentrum: | ± 0,5 mm |
| | Positioniergenauigkeit integral: | ± 0,5 mm |
| Gewicht: | Gewicht Positioniereinheit: | ca. 350 kp |

Die Mechanik für beide Rotationen um eine vertikale Achse 11 und eine horizontale Achse 10 und für die horizontalen Translationen befindet sich unter der Sitzfläche und beansprucht in dieser Ausführungsform weniger als 35 cm Höhe. Gekippt wird die Vorrichtung um eine raumfeste horizontale Achse 10 quer zur Strahlrichtung C. Der Patientenstuhl 21 wird dabei in den Bogenführungen 15 bewegt. Drehung und Kippung sind konzentrisch, wobei der Schnittpunkt der Achsen 11 und 10 raumfest und über die translatorischen Einstellungen in X-, Y- und Z-Richtung in das Isozentrum gelegt werden kann. Mit dem Verfahrbereich der Translationen läßt sich jeder Zielpunkt des Patientenkopfes in das Isozentrum einstellen. Der Verfahrbereich der vertikalen Translation, der sich hinter der Stuhllehne befindet, deckt zusätzlich noch den Patientengrößenausgleich ab. Alle Freiheitsgrade dieser Vorrichtung in der Ausführungsform der Fig. 1 werden elektromotorisch gesteuert.

Sowohl für die integrale Positioniergenauigkeit als auch für die Lage des Isozentrums im Raum wird eine Toleranzgrenze von ±0,5 mm erreicht.

Eine wesentliche Eigenschaft des Behandlungsstuhles ist es, daß eine analoge Positioniertechnik angewendet wird, wie sie bereits bei Patientenliegen erprobt ist. Dazu wird die Drehachse senkrecht gestellt und der Drehwinkel auf 0°, so daß der Patient in die Strahlrichtung schaut. Dann werden mit Hilfe eines Zielgerätes durch drei Translationen in X-, Y- und Z-Richtung die stereotaktischen Koordinaten eingestellt, und zum Schluß werden die Drehwinkel und der Kippwinkel eingestellt, um die Einschußrichtung des Ionenstrahls festzulegen.

Der Vorteil dieser Vorgehensweise ist, daß sie analog zur Positionierung einer Patientenliege abläuft und somit keine erhöhten Schwierigkeiten bei der Bestrahlungsplanung auftreten. Somit bleiben die Änderungen für die Bestrahlungsplanung überschaubar, da keine winkelabhängigen Translationen erforderlich sind. Ein wesentlicher Unterschied der erfindungsgemäßen Vorrichtung ist jedoch die Möglichkeit, mindestens zwei Winkel einstellen zu können. In einem Planungsprogramm wird wegen des horizontalen Strahlrohres des Ionenstrahls bei Bestrahlung mit der Patientenliege mit einem Gantrywinkel von 90° gerechnet, und innerhalb einer Frontalebene kann die Einstrahlrichtung bzw. die Einschußrichtung mit dem Tischwinkel eingestellt werden.

Diesem Planungsprogramm der Patientenliege entspricht eine Verwendung des Patientenstuhles ohne Einsatz der Kippung, so daß Einstrahlrichtungen und Einschußwinkel lediglich innerhalb der transversalen Ebene ausgeführt werden. Bei Verwendung des Kippwinkels gibt es jedoch keine Zuordnung der zwei möglichen Patientenstuhlwinkel zu dem Einstellwinkel einer Patientenliege. Die einzustellenden Stuhlwinkel werden kann mit Hilfe einer Koordinatentransformation aus den Planungswinkeln berechnet. Für die Planungspraxis ist mit dieser Vorrichtung zur Plazierung eines Patienten auf einem Patientenstuhl der entscheidende Vorteil verbunden, daß mit dem gleichen Programm sowohl Pläne für eine Patientenliege als auch für einen Behandlungsstuhl berechnet werden können.

Somit sind im Prinzip die Voraussetzungen für Bestrahlungspläne mit gemischten Feldern (Liege und Stuhl) bei zusätzlichem Einsatz der erfindungsgemäßen Vorrichtung gegeben. Dieses vervielfacht die planerische Freiheit für die Behandlung von Tumor-Patienten mit Tumoren im Kopf- und Halsbereich. Der erfindungsgemäße Patientenstuhl ist deshalb eine Erweiterung von herkömmlichen medizinischen Bestrahlungseinrichtungen und eine Verbesserung der Behandlungsmöglichkeiten von Tumoren im Kopf- und Halsbereich eines Patienten.

Wegen der vorgegebenen geringen Höhe des Bestrahlungspunktes im Bestrahlungsraum über dem Boden des Bestrahlungsraumes sind herkömmliche Patientenstühle ungeeignete, zumal für die Patientenliege bereits die Antriebsmechanik und Antriebseinheit 20 im Zwischenboden unterhalb des Bestrahlungspunktes angeordnet sind. Mit der durch die Anordnung des gesamten Patientenstuhls 21 auf der freihängende, über die Antriebseinheit 20 der Liege ragenden Plattform ergibt sich der besondere Vorteil, daß die Positioniereinrichtung auf Gleitschienen mit guter Reproduzierbarkeit zwischen Behandlungsposition und Parkposition hin- und hergefahren werden kann.

### Bezugszeichenliste

- 1: Tumor-Patient
- 2: Tumor
- 3: Kopf-Halsbereich
- 4: Bestrahlungsmaske
- 5: Ionentherapieraum
- 6: Ionenstrahl
- C: Ionenstrahlrichtung
- 7: Ablenkungsmagnete
- 8: Vorrichtung zum Plazieren eines Patienten
- 9: Isozentrum
- B: Kippbewegung
- 10: horizontale Achse
- A: Drehbewegung um eine vertikale Achse
- 11: vertikale Achse
- 12: Antrieb der Drehbewegung um die horizontale Achse
- 13: Antriebe der Drehbewegung um die vertikale Achse
- 14: Sitzposition
- 15: Bogenführungen
- 16: Verfahrschiene
- 17: Sitzlehne
- 18, 19: Unterbodenführungen
- 20: Antriebseinheit der Patientenliege
- 21: Patientenstuhl
- 22: umgebendes Gewebe
- D: Drehbewegungsrichtung der Patientenliege
- 23: Vorrichtung zum Verfahren des Patientenstuhls

- 24: Plattform
- 25: Antriebseinheit für die X-Richtung
- 26: Antriebseinheit für die Y-Richtung
- 27: Antriebseinheit für die Höheneinstellung
- 28: Säule

## Patentansprüche

1. Vorrichtung zum Plazieren eines in sitzender Haltung fixierten Tumor-Patienten (1) mit Mechanismen, die den Tumor des Patienten unter den Bewegungsfreiheitsgraden der Vorrichtung (21) im Isozentrum (9) eines Strahls leichter Ionen (6) aus einer unter Raumkoordinaten unveränderlichen Richtung (C) hält, welcher orthogonal zur Strahlrichtung in horizontaler und vertikaler Richtung über den Tumorquerschnitt geführt wird,
wobei zum Plazieren des fixierten Patienten mit Positionieren des Tumors im Isozentrum (9) des Ionenstrahls (6) eine translatorische Vorstellung in X-, Y- und Z-Richtung vorgesehen ist und
wobei zum Halten des Tumors in dem Isozentrum (9) des Ionenstrahls (6) eine Drehbewegung (A, B) der Vorrichtung (21) um eine vertikale Achse (11) und eine horizontale Achse (10) vorgesehen ist, die den Ionenstrahl (6) für alle Positionen in X-, Y- und Z-Richtung im Isozentrum schneiden.

2. Vorrichtung nach Anspruch 1, **dadurch gekennzeichnet, dass** Antriebe (12, 13) für Drehbewegungen um eine horizontale und eine vertikale Achse (10, 11) mit ihren Schnittpunkten in dem Isozentrum (9) des Ionenstrahls (5) unter der Sitzposition (14) des Patienten (1) angeordnet sind.

3. Vorrichtung nach Anspruch 1 oder 2, **dadurch gekennzeichnet, dass** die Vorrichtung (8) zur Drehung oder Kippung um eine horizontale Achse (10) Bogenführungen (15) unter der Sitz-und/oder Liegefläche aufweiset.

4. Vorrichtung nach einem der vorhergehenden Ansprüche, **dadurch gekennzeichnet, dass** alle verstellbaren Bewegungsfreiheitsgrade elektromotorisch einstellbar sind.

5. Vorrichtung nach einem der vorhergehenden Ansprüche, **dadurch gekennzeichnet, dass** die Anordnung der Bewegungsfreiheitsgrade der Vorrichtung (8) in der Reihenfolge von unten nach oben Kippung, Rotation, Translation (X, Y, Z) eine isozentrische Kippung und Rotation bewirken.

6. Vorrichtung nach einem der vorhergehenden Ansprüche, **dadurch gekennzeichnet, dass** die Vorrichtung (8) zum Verschieben in X-, Y-und Z-Richtung, den drei Translationen der stereotaktischen Koordinaten und zum Verdrehen um eine horizontale und eine vertikale Achse (10, 11) als Antriebsaggregate (12,13) Schrittmotoren mit Positionsmessern, Endschaltern und elektronischen Steuermodulen aufweist.

7. Vorrichtung nach einem der vorhergehenden Ansprüche, **dadurch gekennzeichnet, dass** die in der Parkposition befindliche Vorrichtung eine Bestrahlung auf einer Patientenliege in Bestrahlungsposition freigibt.

8. Vorrichtung nach einem der vorhergehenden Ansprüche, **dadurch gekennzeichnet, dass** die Antriebe für translatorische Verschiebungen der Vorrichtung (8) ausserhalb einer unmittelbaren Sitzposition (14) der Vorrichtung (8) angeordnet sind.

9. Vorrichtung nach einem der vorhergehenden Ansprüche, **dadurch gekennzeichnet, dass** die Vorrichtung (8) mit einer Not-Aus-Schaltungsautomatik ausgestattet ist.

10. Vorrichtung nach einem der vorhergehenden Ansprüche, **dadurch gekennzeichnet, dass** die Vorrichtung (8) mittels eines Programmes steuerbar ist, das einen Kollisionsschutz vorsieht und mit einer Bewegungsgrenz-Überwachungseinrichtung zusammenwirkt.

11. Vorrichtung nach einem der vorhergehenden Ansprüche, **dadurch gekennzeichnet, dass** eine translatorische Verschiebung der Vorrichtung (8) in Richtung des Ionenstrahls (6) auf Verfahrschienen (16) vorgesehen ist, wobei ein grosser Verfahrweg der Vorrichtung (8) von einer Parkposition in eine Patientenbehandlungsposition und eine Feineinstellvorrichtung in der Patientenbehandlungsposition vorgesehen sind.

12. Vorrichtung nach einem der vorhergehenden Ansprüche, **dadurch gekennzeichnet, dass** die Positionsgenauigkeit der Vorrichtung (8) in allen translatorisch verstellbaren Freiheitsgraden (X, Y, Z) kleiner oder gleich 0,5 mm ist.

13. Vorrichtung nach einem der vorhergehenden Ansprüche, **dadurch gekennzeichnet, dass** die Vorrichtung (8) mit einer Genauigkeit von ± 0,1 bis ± 0,5 mm in dem Isozentrum (9) einstellbar ist.

14. Vorrichtung nach einem der vorhergehenden Ansprüche, **dadurch gekennzeichnet, dass** die Vorrichtung (8) mit einem Positionskontrollgerät im Ionenbehandlungsraum (4) zusammenwirkt, das die Tumorposition im Isozentrum (9) Über wacht.

15. Vorrichtung nach Anspruch 14, **dadurch gekennzeichnet, dass** das Positionskontrollgerät eine Röntgenstrahlkamera ist.

16. Vorrichtung nach einem der vorhergehenden Ansprüche, **dadurch gekennzeichnet, dass** die Vorrichtung (8) einen Rechner aufweist, der wahlweise die Zielkoordinaten und Behandlungseinstellungen für eine Plazierung des Patienten (1) in liegender und/oder sitzender Position umrechnet.

17. Vorrichtung nach einem der vorhergehenden Ansprüche, **dadurch gekennzeichnet, dass** die Zielkoordinaten für die Bestrahlung eines Tumors im Kopf-Halsbereich durch die Translationsbewegunen und anschliessender isozentrischer Rotation und/oder isozentrischer Kippung einstellbar sind.

18. Vorrichtung nach einem der vorhergehenden Ansprüche, **dadurch gekennzeichnet, dass** zur Überwachung der Bestrahlung des Patienten Kameraköpfe der PET-Kamera um die Ionenstrahlachse (6) drehbar gelagert sind.

19. Vorrichtung nach einem der vorhergehenden Ansprüche, **dadurch gekennzeichnet, dass** die Vorrichtung (8) in Verbindung mit einer Patientenliege eine kombinierte Bestrahlung eines Patienten in sitzender und liegender Haltung ermöglicht.

20. Vorrichtung nach einem der vorhergehenden Ansprüche, **dadurch gekennzeichnet, dass** die Vorrichtung (8) eine Höhenverstelleinrichtung zur Anpassung der Vorrichtung (8) an die Körpergrösse des Patenten(1) in bezug auf das Isozentrum (9) des Ionenstrahls (6) aufweist.

21. Vorrichtung nach Anspruch 20, **dadurch gekennzeichnet, dass** die Höhenverstelleinrichtung einen Verfahrbereich von ± 100 bis ± 500 mm, vorzugsweise ± 200 bis ± 300 mm, aufweist,

22. Vorrichtung nach Anspruch 20 oder Anspruch 21, **dadurch gekennzeichnet, dass** die Höhenverstelleinrichtung eine Verfahrgeschwindigkeit von 1 bis 15 mm/s, vorzugsweise 2 bis 5 mm/s, aufweist.

23. Vorrichtung nach einem der vorhergehenden Ansprüche, **dadurch gekennzeichnet, dass** die Vorrichtung (8) in einer Patientenbehandlungsposition in den horizontalen Translationsverschiebungen in X-und Y-Richtung einen Verfahrbereich von ± 100 bis ± 200 mm, vorzugsweise von ± 120 bis ± 150 mm, aufweist.

24. Vorrichtung nach Anspruch 23, **dadurch gekennzeichnet, dass** die Verfahrgeschwindigkeit der Vorrichtung (8) in X- und Y-Richtung zwischen 5 und 20 mm/s, vorzugsweise zwischen 8 und 10 mm/s, liegt.

25. Vorrichtung nach einem der vorhergehenden Ansprüche, **dadurch gekennzeichnet, dass** die Vorrichtung (8) zur Drehung um eine horizontale Achse (10) eine Kippbewegungsvorrichtung für einen Kippbereich von ± 30°, vorzugsweise ± 24°, aufweist.

26. Vorrichtung nach Anspruch 25, **dadurch gekennzeichnet, dass** die Geschwindigkeit der Kippbewegung (B) zwischen 0,5 bis 1°/s, vorzugsweise zwischen 0,6 bis 0,8°/s, liegt.

27. Vorrichtung nach einem der vorhergehenden Anspruche, **dadurch gekennzeichnet, dass** die Vorrichtung (8) zur Drehung um eine vertikale Achse (11) einen Drehbereich von 0 bis 360° aufweist.

28. Vorrichtung nach Anspruch 27, **dadurch gekennzeichnet, dass** die Drehgeschwindigkeit um die vertikale Achse (11) zwischen 1 bis 10°/s, vorzugsweise zwischen 3 bis 6°/s, liegt.

## Claims

1. Device for positioning a tumour patient (1) being fixed in the seated position, including mechanisms which hold the tumour of the patient with respect to the degrees of freedom of movement of the device (21) in the isocentre (9) of a light ion beam (6) from an unchangeable direction (C) that is fixed by spatial coordinates,
which beam being guided over the tumour cross-section in the horizontal and vertical direction orthogonally to the beam direction,
wherein for positioning the fixed patient with aligning the tumour in the isocentre (9) of the ion beam (6), a translational displacement in X, Y and Z direction is provided, and
wherein for holding the tumour in the isocentre (9) of the ion beam (6), a rotational movement (A, B) of the device (21) around a vertical axis (11) and a horizontal axis (10) is provided, which intersect the ion beam (6) in the isocentre for all positions in X, Y and Z direction.

2. Device according to claim 1, **characterised in that** drives (12, 13) for rotational movements about a horizontal and a vertical axis (10, 11) with their points of intersection in the isocentre (9) of the ion beam (5) are arranged below the seat position (14) of the patient (1).

3. Device according to claim 1 or 2, **characterised in that,** for the rotation or tilting about a horizontal axis (10), the device (8) has curved guides (15) below the seat area and/or couch area.

4. Device according to any one of the preceding claims, **characterised in that** all adjustable degrees of freedom of movement can be set by electric motors.

5. Device according to any one of the preceding claims, **characterised in that** the arrangement of the degrees of freedom of movement of the device (8) bring about isocentric tilting and rotation in the order, from bottom to top, tilting, rotation, translation (X, Y, Z).

6. Device according to any one of the preceding claims, **characterised in that** the device (8) has, as drive units (12, 13) for displacement in the X, Y and Z directions, the three translations of the stereotactic coordinates, and for rotation about a horizontal and a vertical axis (10, 11), stepper motors having position-measuring means, limit switches and electronic control modules.

7. Device according to any one of the preceding claims, **characterised in that,** when located in the park position, the device permits irradiation on a patient couch that is in the irradiation position.

8. Device according to any one of the preceding claims, **characterised in that** the drives for translational displacements of the device (8) are arranged outside an immediate seat position (14) of the device (8).

9. Device according to any one of the preceding claims, **characterised in that** the device (8) is provided with an automatic emergency disconnector switch.

10. Device according to any one of the preceding claims, **characterised in that** the device (8) is controllable by means of a program that provides collison protection and cooperates with a movement-limiting/monitoring means.

11. Device according to any one of the preceding claims, **characterised in that** a translational displacement of the device (8) is provided in the direction of the ion beam (6) on travel rails (16), a long path of travel of the device (8) from a park position into a patient treatment position being provided, and a device for fine adjustment in the patient treatment position being provided.

12. Device according to any one of the preceding claims, **characterised in that** the positioning accuracy of the device (8) in all translationally adjustable degrees of freedom (X, Y, Z) is less than or equal to 0.5 mm.

13. Device according to any one of the preceding claims, **characterised in that** the device (8) can be set in the isocentre (9) with an accuracy of from ±0.1 to ±0.5 mm.

14. Device according to any one of the preceding claims, **characterised in that** the device (8) cooperates with a position-monitoring means in the ion treatment room (4), which means monitors the tumour position in the isocentre (9).

15. Device according to claim 14, **characterised in that** the position-monitoring means is an X-ray camera.

16. Device according to any one of the preceding claims, **characterised in that** the device (8) has a computer that recalculates, as desired, the target coordinates and treatment settings for positioning the patient (1) in the lying and/or seated positions.

17. Device according to any one of the preceding claims, **characterised in that** the target coordinates for the irradiation of a tumour in the head/neck region are adjustable by the translational movements and subsequent isocentric rotation and/or isocentric tilting.

18. Device according to any one of the preceding claims, **characterised in that** camera heads of the PET camera are rotatably mounted about the ion beam axis (6) for monitoring the irradiation of the patient.

19. Device according to any one of the preceding claims, **characterised in that** the device (8) in conjunction with a patient couch renders possible a combined irradiation of a patient in the seated and lying positions.

20. Device according to any one of the preceding claims, **characterised in that** the device (8) has a height-adjusting means for matching the device (8) to the body size of the patient (1) in relation to the isocentre (9) of the ion beam (6).

21. Device according to claim 20, **characterised in that** the height-adjusting means has a travel range of from ±100 to ±500 mm, preferably from ±200 to ±300 mm.

22. Device according to claim 20 or claim 21, **characterised in that** the height-adjusting means has a travel speed of from 1 to 15 mm/s, preferably from 2 to 5 mm/s.

23. Device according to any one of the preceding claims, **characterised in that,** in a patient treatment position, the device (8) has, in the horizontal translational displacements in the X and Y directions, a travel range of from ±100 to ±200 mm, preferably from ±120 to ±150 mm.

24. Device according to claim 23, **characterised in that** the travel speed of the device (8) in the X and Y directions is from 5 to 20 mm/s, preferably from 8 to 10 mm\s.

25. Device according to any one of the preceding claims, **characterised in that,** for rotation about a horizontal axis (10), the device (8) has a tilting movement means for a tilting range of ±30°, preferably ±20°.

26. Device according to claim 25, **characterised in that** the speed of the tilting movement (B) is from 0.5 to 1 °/s, preferably from 0.6 to 0.8 °/s.

27. Device according to any one of the preceding claims, **characterised in that,** for rotation about a vertical axis (11), the device (8) has a rotation range of from 0 to 360°.

28. Device according to claim 27, **characterised in that** the speed of rotation about the vertical axis (11) is from 1 to 10 °/s, preferably from 3 to 6 °/s.

## Revendications

1. Dispositif de mise en place d'un patient (1) atteint d'une tumeur immobilisé en position assise avec des mécanismes, qui maintient la tumeur du patient sous les degrés de liberté de mouvement du dispositif (21) dans l'isocentre (9) d'un faisceau d'ions légers (6) à partir d'une direction (C) constante sous des coordonnées spatiales, lequel est guidé de façon orthogonal par rapport à la direction du faisceau en direction horizontale et verticale sur la coupe transversale de la tumeur,
un réglage de translation dans la direction X, Y et Z étant prévu pour positionner le patient immobilisé avec la tumeur dans l'isocentre (9) du faisceau d'ions (6) et
pour maintenir la tumeur dans l'isocentre (9) du faisceau d'ions (6) un mouvement de rotation (A, B) du dispositif (21) étant prévu autour d'un axe vertical (11) et d'un axe horizontal (10) qui coupent le faisceau d'ions (6) pour toutes les positions dans une direction X, Y et Z dans l'isocentre.

2. Dispositif selon la revendication 1,
**caractérisé en ce que** des mécanismes d'entraînement (12, 13) pour des mouvements de rotation sont disposés autour d'un axe horizontal et vertical (10, 11) avec leurs intersections dans l'isocentre (9) du faisceau d'ions (5) sous la position du siège (14) du patient (1).

3. Dispositif selon la revendication 1 ou 2,
**caractérisé en ce que** le dispositif (8) comprend des guidages en courbe (15) sous la surface d'assise et/ou de couchage pour effectuer une rotation ou une inclinaison autour d'un axe horizontal (10).

4. Dispositif selon l'une des revendications précédentes,
**caractérisé en ce que** tous les degrés de liberté de mouvement réglables peuvent être réglés de façon électromotrice.

5. Dispositif selon l'une des revendications précédentes, **caractérisé en ce que** la disposition des degrés de liberté de mouvement du dispositif (8) dans l'ordre de bas en haut de l'inclinaison, la rotation à la translation (X, Y, Z) provoque une inclinaison et une rotation isocentrique.

6. Dispositif selon l'une des revendications précédentes,
**caractérisé en ce que** le dispositif (8) comprend comme mécanismes d'entraînement (12, 13) pour la translation dans la direction X, Y et Z, les trois translations des coordonnées stéréotactiques et pour une rotation autour d'un axe horizontale et vertical (11, 12) des moteurs pas à pas avec des mesureurs de position, des interrupteurs de fin de course et des modules de commande électroniques.

7. Dispositif selon l'une des revendications précédentes,
**caractérisé en ce que** le dispositif présent en position d'arrêt fixe libère une irradiation sur l'assise du patient en position d'irradiation.

8. Dispositif selon l'une des revendications précédentes, **caractérisé en ce que** les mécanismes d' entraînement pour des déplacements par translation du dispositif (8) sont disposés en dehors d'une position assise (14) immédiate du dispositif (8)

9. Dispositif selon l'une des revendications précédentes, **caractérisé en ce que** le dispositif (8) est équipé d'un dispositif automatique d'arrêt d'urgence.

10. Dispositif selon l'une des revendications précédentes, **caractérisé en ce que** le dispositif (8) peut être commandé à l'aide d'un programme qui prévoit une protection anticollision et qui fonctionne conjointement avec un dispositif de surveillance de limitation de mouvement.

11. Dispositif selon l'une des revendications précédentes,
**caractérisé en ce qu'**un déplacement par translation du dispositif (8) en direction du faisceau d'ions (6) est prévu sur les rails de déplacement (16), une grande course de déplacement du dispositif (8) étant prévue depuis une position d'arrêt fixe jusqu'à une position de traitement du patient et un dispositif de réglage de précision dans la position de traitement du patient.

12. Dispositif selon l'une des revendications précédentes,
**caractérisé en ce que** la précision de la position du dispositif (8) dans tous les degrés de libertés de translation réglables (X, Y, Z) est inférieure ou égale à 0,5 mm.

13. Dispositif selon l'une des revendications précédentes,
**caractérisé en ce que** le dispositif (8) peut être réglé avec une précision de ± 0, 1 à ± 0, 5 mm dans l'isocentre (9).

14. Dispositif selon l'une des revendications précédentes, **caractérisé en ce que** le dispositif (8) coopère avec un dispositif de contrôle de position dans la zone de traitement d'ions (4) qui surveille la position de la tumeur dans l'isocentre (9).

15. Dispositif selon la revendication 14, **caractérisé en ce que** le dispositif de contrôle de position est une caméra à faisceaux X.

16. Dispositif selon l'une des revendications précédentes,
**caractérisé en ce que** le dispositif (8) comprend ainsi un ordinateur qui convertit au choix les coordonnées cibles et les réglages de traitement pour un placement du patient (1) en position allongée et/ou assise.

17. Dispositif selon l'une des revendications précédentes,
**caractérisé en ce que** les coordonnées cibles pour l'irradiation d'une tumeur dans la région de la tête et du cou peuvent être réglées par les mouvements de translation et la rotation isocentrique et/ou inclinaison isocentrique qui s'ensuivent.

18. Dispositif selon l'une des revendications précédentes,
**caractérisé en ce que,** pour surveiller l'irradiation du patient, des têtes de caméra de la caméra TEP sont logées de façon pivotante autour de l'axe de faisceau d'ions (6).

19. Dispositif selon l'une des revendications précédentes,
**caractérisé en ce que** le dispositif (8) en relation avec la couchette pour le patient permet une irradiation combinée d'un patient en position assise et allongée.

20. Dispositif selon l'une des revendications précédentes,
**caractérisé en ce que** le dispositif (8) comprend un dispositif de réglage de hauteur servant à adapter le dispositif (8) à la taille du patient (1) par rapport à l'isocentre (9) du faisceau d'ions (6).

21. Dispositif selon la revendication 20,
**caractérisé en ce que** le dispositif de réglage de hauteur comprend une zone de déplacement de ± 100 à ± 500 mm, de préférence ± 200 à + 300 mm.

22. Dispositif selon la revendication 20 ou la revendication 21, **caractérisé en ce que** dispositif de réglage de hauteur présente une vitesse de déplacement de 1 à 15 mm/s, de préférence, de 2 à 5 mm/s.

23. Dispositif selon l'une des revendications précédentes, **caractérisé en ce que** le dispositif (8) dans une position de traitement du patient présente une zone de déplacement de ± 100 à ± 200 mm, de préférence de ± 120 à ± 150 mm, pour les déplacements par translation horizontaux dans la direction X et Y.

24. Dispositif selon la revendication 23, **caractérisé en ce que** la vitesse de déplacement du dispositif (8) dans la direction X et Y se situe entre 5 et 20 mm/s, de préférence entre 8 et 10 mm/s.

25. Dispositif selon l'une des revendications précédentes,
**caractérisé en ce que** le dispositif (8) comprend, pour pivoter autour d'un axe horizontal (10), un dispositif de mouvement d'inclinaison pour une zone d'inclinaison de ± 30°, de préférence de ± 20°.

26. Dispositif selon la revendication 25, **caractérisé en ce que** la vitesse du mouvement d'inclinaison (B) se situe entre 0,5 et 1°/s, de préférence entre 0,6 et 0,8°/s.

27. Dispositif selon l'une des revendications précédentes, **caractérisé en ce que** le dispositif (8) comprend, pour pivoter autour d'un axe vertical (11), une zone de rotation de 0 à 360°.

28. Dispositif selon la revendication 27, **caractérisé en ce que** la vitesse de rotation autour de l'axe vertical (11) se situe entre 1 et 10°/s, de préférence entre 3 et 6°/s.
